# EUROPEAN PATENT SPECIFICATION

(11) **EP 3 548 017 B1**
(45) Date of publication and mention of the grant of the patent: **17.03.2021**
(21) Application number: 17826550.0
(22) Date of filing: 29.11.2017
(51) Int. Cl.: A61K 31/195, A61K 31/20, A61K 31/351, A61K 36/63, A61P 17/14, A61Q 7/00

(54) **COMPOSITION COMPRISING GLUTATHION TIOESTER AND SECOIRIDOIDS OF THE PLANT OLEA EUROPEA FOR TREATING HAIR LOSS**
ZUSAMMENSETZUNG ENTHALTEND GLUTATHION TIOESTER UND SECOIRIDOIDE AUS DER PFLANZ OLEA EUROPEA ZUR BEHANDLUNG VON HAARAUSFALL
COMPOSITION CONTENANT GLUTATHIONE TIOESTER ET SECOIRIDOIDES DE LA PLANTE OLEA EUROPEA POUR TRAITER LA PERTE DE CHEVEUX ET DE POILS

(30) Priority: 29.11.2016 IT 201600120528
(43) Date of publication of application: 09.10.2019
(73) Proprietor: Innbiotec Pharma S.R.L., 52100 Arezzo (IT)
(72) Inventor: LIGURI, Gianfranco, 50141 Firenze (IT); NEVISCHI, Silvio, 51010 Uzzano (PT) (IT); SERNESI, Ilaria, 50012 Bagno a Ripoli (FI) (IT)
(74) Representative: Martini, Riccardo
(86) International application number: PCT/IB2017/057500
(87) International publication number: WO 2018/100510

(56) References cited:
- EP-A1- 0 656 201
- WO-A1-2013/068964
- WO-A1-2014/010900
- PENSALFINI ET AL: "Protective effect of new S-acylglutathione derivatives against amyloid-induced oxidative stress", FREE RADICAL BIOLOGY AND MEDICINE, ELSEVIER INC, US, vol. 44, no. 8, 9 February 2008 (2008-02-09), pages 1624-1636, XP022575894, ISSN: 0891-5849

## Description

### Technical Field

This invention relates to a composition containing conjugates of glutathione with carboxylic acids in association with polyphenols of *Europea olea.* The formulation and method described by this invention can be used in the form of a pharmaceutical remedy, a cosmetic preparation, a medical device or a food supplement, in order to prevent or treat hair loss or to promote hair growth.

### Background Art

Hair loss, or alopecia, in the human species is a common phenomenon which affects both men and women. There are six main types of hair loss, of which the most common is androgenetic alopecia, or male pattern baldness, which represents over 95% of all variants. Androgenetic alopecia is prevalent in men, but equally affects women of postmenopausal age, and can affect 75% of women over the age of 65 (https://www.nlm.nih.gov/medlineplus /ency/article/003246.htm).

At present, remedies for treating hair loss are classified as drugs, medical devices or cosmetics. The most frequently used drugs for this purpose are based on the active ingredient Finasteride, which inhibits 5-α-reductase, and Minoxidil, a vasodilator drug that improves the micro-circulation of the scalp. Both are effective at reducing short-term hair loss, but are accompanied by side effects, including impotence, male sexual dysfunction and gynaecomastia, pruritus, rashes and tachycardia.

The root of the hair is enclosed within the hair follicle, underneath the surface of the skin. At the base of the follicle is the dermal papilla, which is fed by the blood flow, which provides nourishment to stimulate the production of new hair. The dermal papilla plays a fundamental role in dermal-epidermal interactions and is of great importance for the construction of the hair and the growth cycle (Bernard BA. Hair biology: an update. Int J Cosmet Sci. 2002. 24(1): 13-6).

Hair production occurs at different stages, including active growth (anagen), cessation (catagen) and resting (telogen). Anagen is the phase of active follicular growth, and has a variable duration of between 3 and 6 years, during which the root of the hair divide rapidly, adding itself to the hair shaft. Normally 80-90% of all hair is in the anagen phase. The catagen phase is a short transition phase that signals the end of active hair growth, affects about 1% of all hair and is characterised by slower metabolism and reduced root growth. When hair loss is in progress, a shorter anagen phase and a longer catagen phase result in a reduction of the percentage of hair in the anagen phase.

The catagen phase is followed by the telogen phase, the resting phase of the hair follicle which affects about 4-14% of the total hair. In the telogen phase, the hair follicle contracts gradually and the root of the hair is pushed upwards, until it is eventually removed.

At the beginning of a new anagen phase the hair bulbs, which are surrounded by hair follicles in the anagen phase, combine with the hair's papilla to induce the formation of a new hair, which pushes upwards and naturally remove the old hair in the telogen phase (Schneider MR, Schmidt-Ullrich R, Paus R. The hair follicle as a dynamic mini-organ. Curr Biol. 2009; 19: R132-R142).

Based on this knowledge, the objectives for treating alopecia include conversion of telogen hair follicles to anagen hair follicles, and prolongation of the anagen phase.

Hair loss is often the only true symptom of incipient baldness. On the other hand, the more conspicuous it is, the less it corresponds, in almost all cases, to a real danger of baldness. The fall of a few hundred, sometimes thousands, of hair a day defines the phenomenon of effluvium, an impressive event, often a source of anxiety and despair, but generally benign, self-limited, reversible (Marliani A., www.sitri.it/terapia. doc). Among the common causes of effluvium in the telogen phase (*telogen effluvium*) are: febrile diseases, childbirth, acute physical stress, chronic systemic diseases (neoplasms, cirrhosis, Hodgkin's M., tuberculosis, lue, leukaemia, etc.), surgical procedures under general anaesthesia, severe psychological stress, hypothyroidism, neoplasia, drugs ( allopurinol, anticonvulsants, anti-thyroid drugs, beta-blockers, warfarin, heparin, lithium, dicumarolics, vitamin A, certain oral contraceptives etc ...). Among the common causes of effluvium in the anagen phase (*anagen effluvium*) are: anti-neoplastic chemotherapy, ionizing radiation, strong thermal increases, aprotoxic diets, poisonings, drugs (arsenic, bismuth, colchicine, thallium, beta-blockers).

Effluvium is considered to be a benign condition with strong hair loss, generally reversible: it does not correspond, therefore, to a genuine loss of hair.

Defluvio, on the other hand, is characterised by hair loss of a dystrophic appearance, in the telogen phase. The hair that falls out is short, thin, with bulbs of reduced diameter, and their loss often indicates a definitive loss.

The temporal evolution of alopecia is characterised by a cascade of irreversible phenomena. Firstly, a progressive involution and miniaturisation of the hair follicle is observed. Then, the follicle moves from the subcutaneous layer to the superficial layers of the dermis, giving rise to a smaller and thinner hair shaft. Subsequently, fibrosis of the connective tissue around the bulb takes place. This is often accompanied by an inflammatory process. After the onset of these changes to hair follicles and the surrounding tissue, hair loss and irreversible bulb atrophy occur.

With advancing age, the production of free radicals increases, while physiological antioxidant defence mechanisms decline. This imbalance leads to the progressive damage of cellular structures, which is likely to underlie the characteristic phenotype of ageing. Hair ageing manifests itself as a decrease in the function of melanocytes, or greying, and a reduced production of hair, or alopecia. The results of recent research support the hypothesis of oxidative stress as a key mechanism which contributes to hair grafting and hair loss (Int J Trichology. 2009 Jan-Jun; 1 (1): 6-14; J Invest Dermatol. 2015 May; 135 (5): 1244-52;). Solutions that can combat oxidative stress could open new strategies for preventing and treating the hair greying process and alopecia linked to ageing.

By analysing the etiology and evolutionary mechanisms of alopecia, with the results of experimental and clinical studies, it is clear to experts in the field that hair loss is characterised by inflammation and oxidative stress at the level of the hair bulb. EP0656201A1 describes a method for preventing hair loss and stimulating the growth of new hair, a method that involves the use of glutathione esters (monoesters or diethyl esters in particular) as stimulators of intracellular glutathione synthesis.

Recently, a class of glutathione derivatives with carboxylic acids has been shown to be effective in counteracting intra-cellular oxidative stress (Free Radical Biology & Medicine 44 (2008) 1624-1636). Their synthesis, anti-radical activity and anti-ageing effects are described in WO2013068964A1 and ITPO2013A000001. The presence of a hydrophobic carboxylic acid molecule, linked to the thiol group of glutathione, has two useful effects: i) it protects it from oxidation, preserving the active form of glutathione; ii) it allows it to cross cell membranes and inactivate reactive oxygen species at an intra-cellular level. Thioesters of glutathione with polyunsaturated fatty acids have been shown to be particularly effective at counteracting oxidative stress (Photochemistry and Photobiology, 2013, 89: 442-452). These derivatives of glutathione with carboxylic acids are, for these reasons, forms with high glutathione bioavailability.

Recently, a team of South Korean researchers has published a study into the efficacy of subcutaneously injected oleuropein preparations during the transition from the telogen to anagen phase (Tong T, Kim N, Park T. Topical Application of Oleuropein Induces Anagen Hair Growth in Telogen Mouse Skin. PLoS One. 2015 Jun 10; 10 (6): e0129578). The observed effect was significantly greater than that obtained with Minoxidil (WO 2014/010900 A1).

### Disclosure of Invention

This invention provides a solution, in the form of a pharmaceutical, nutraceutical or medical device, for counteracting hair loss or promoting its regrowth. The solution provided by this invention is based on the synergy between the free radical inactivation activity exerted by the thioesters of glutathione, and the anti-inflammatory activity exerted by the secoiridoids of *European olea.*

According to this invention, the composition that is the subject of the invention can be used to prepare a pharmaceutical formulation, a pharmaceutical remedy, a cosmetic product, a medical device, a nutraceutical preparation or a functional food for preventing or treating hair loss.

The combination, in the same preparation, of a component that is highly effective at combatting oxidative stress and a component capable of fighting the inflammatory process - both alterations that are characteristically found in in the hair bulbs of skin in alopecia - has shown itself to be surprisingly superior to a simple additive effect of both components.

Being two components of bioavailable derivatives of glutathione and oleuropein respectively, the unexpected synergy of their combination in counteracting hair loss and in stimulating their regrowth, can be ascribed on the one hand, to their shared antioxidant characteristics, and on the other to the proven ability of glutathione to restore redox homeostasis, which is typically altered in the inflammatory process. In view of the effects observed, and on the basis of recent publications about the mechanisms of action involved in their activity, an interaction between these two substances can be suggested in the same biochemical, metabolic and signalling pathways - intracellularly - with the overall effect of strengthening their biological effects (British Journal of Nutrition (2006), 96, 811-819 DOI: 10.1017; Proc. Natl.Acad.Sci.USA 103:13086-13091; 2006.). The bioavailable derivative of glutathione can be obtained from a direct reaction between glutathione and S-acyl-Coenzyme A or via a conjugation reaction between an activated carboxylic acid and glutathione in its reduced form. This latter synthetic route is especially indicated for manufacturing glutathione derivatives suitable for industrial use, and is described in WO2013/068964A1.

Oleuropein can also be obtained by synthesis (e.g., WO96/14064), although the most convenient production method for industrial use is extraction from its natural sources. Particularly useful for this purpose are the leaves of *European olea* (e.g. US2003-0017217) or plant waters produced in the olive oil production process, due to their richness in this secoiridoid polyphenol. The powder obtained by evaporating the solvent from the extract with a hydro-alcoholic solution of these materials and further purification steps can provide variable percentages, of between 5% and 90% of oleuropein, in both glycated and aglycone forms, along with variable proportions of other polyphenols and terpenoids.

The sources reported herein for the production of thioesters of glutathione and oleuropein are reported for the sole purpose of description, and do not exclude any other source or methods for obtaining the two components, for the purposes of this invention. According to this invention, the composition for preventing or treating hair loss can be made in the form of a pharmaceutical formulation, a nutraceutical preparation, a medical device, a cosmetic product. When the composition is prepared as a pharmaceutical remedy, the preferred routes of administration are the oral route or the parenteral route and the composition may comprise a carrier, a lubricant, a humectant, a sweetener, a flavouring agent, an emulsifier and one or more pharmaceutically acceptable preservatives, chosen from those commonly used for these purposes and known to those who are skilled in the art.

When the composition that forms the subject of this invention is prepared as a cosmetic formulation, the preferred method of administration is topical application in the form of liquid lotion, ointment, solution, suspension, emulsion, paste, ointment, gel, or cream, powder, soap or aerosol, and the site of application is the external surface of the scalp or a part thereof. The cosmetic formulation may contain, in addition to the two components responsible for the anti-alopecia activity described herein, substances suitable for promoting their penetration into the lower layers of the skin, such as alcohols or glycols or other organic compounds with a reduced polarity, and other additives such as antioxidants, stabilizers, vasodilators, vitamins, pigments, flavourings and carriers. Said additives can be selected from a multitude of compounds from those familiar to experts in the art of formulating cosmetic products.

When the composition is prepared as a medical device, the preferred mode of administration is the multi-intracutaneous injection (*Micro-needling* or similar techniques), ionophoresis or topical application onto the external surface of the scalp or part thereof, in the form of liquid lotion, ointment, solution, suspension, emulsion, paste, ointment, gel, of cream, powder, soap or aerosol.

When the composition is prepared as a nutraceutical preparation, it may include a carrier, a sweetener, one or more antioxidants, one or more mineral salts, one or more vitamins, one flavouring agent, one emulsifier and one or more preservatives, chosen from amongst those commonly used as food additives and known to those skilled in the art.

In order to prevent hair loss and promote hair growth in the scalp, the simultaneous administration of the two active principles contained in the composition forming the subject of this invention, by both topical and systemic administration, via concomitant administration, has been particularly effective, for a period of not less than one month, for a scalp lotion and a nutritional supplement.

### Detailed Description of the Invention

According to the invention, a pharmaceutical composition for combating hair loss and promoting hair growth in humans and other species of mammals includes, as active ingredients, glutathione thioester and at least one secoiridoid from *Europea olea* in the form of a glycoside or aglycone.

In particular, glutathione thioester consists of one or more S-acyl derivatives of glutathione with the chemical formula: 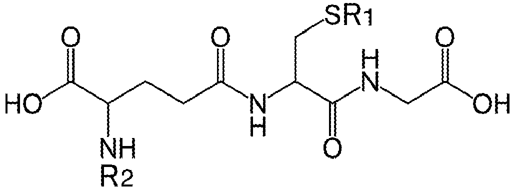 wherein R1 is a carboxylic diacid residue with 2-22 carbon atoms, saturated or unsaturated, and R2 is a hydrogen atom or a carboxylic acid residue with 2-22 carbon atoms, saturated or unsaturated.

The secoiridoid from *Europea olea* in its glycosidic form is glycosylated oleuropein, a compound represented by the chemical formula:

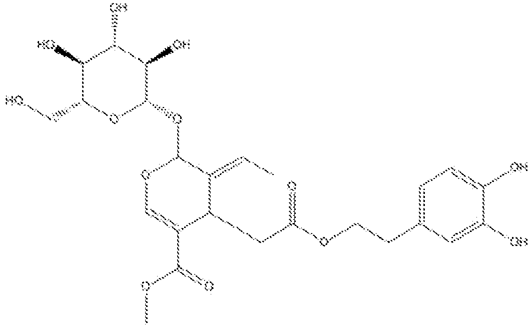

The secoiridoid from *Europea olea* in the form of an aglycone is oleuropein aglycone, a compound represented by the chemical formula:

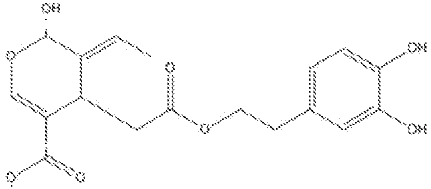

Alternatively, a secoiridoid from *Europea olea* can consist of one or more oleuropein metabolites, e.g. tyrosol or hydroxytyrosol, or oleuroside, elenoic acid, ligstroside and nuzenide.

The secoiridoid from *Europea olea* is typically supplied from a dried hydro-alcoholic extract of *European olea* tissues, preferably from the leaves of *Europea olea.*

Fortunately, glutathione thioester is present in amounts ranging from 1 mg to 500 mg per gram, preferably from 5 mg to 200 mg per g, and the secoiridoid from *Europea olea* is present in amounts ranging from 1 to 350 mg per g, preferably from 10 to 100 mg per g. In a preferred form, in addition to the glutathione thioester and at least one secoiridoid from *Europea olea,* the composition contains other substances that are useful for its efficacy or administration or storage, like a carrier, an antioxidant, a flavouring agent, a pigment, one or more micro-nutrients, an emulsifier, a solvent and a stabiliser. According to this invention, a method, and in particular a cosmetic method, to counteract hair loss and promote hair growth involves using the composition described above, in liquid or solid form, as a pharmaceutical remedy, cosmetic product, medical device, nutraceutical preparation or functional food.

The composition can be administered orally, parenterally, topically, by intradermal micro-injections or by ionophoresis.

In one particularly advantageous method, the two active ingredients are administered simultaneously at both the topical and systemic levels, by using a hair lotion and a nutritional supplement.

A few examples of the invention are detailed below. These examples are for illustrative purposes.

### Example 1

A hydro-alcoholic lotion, having the following composition (active preparation 1): Ethanol 48.0% (v/v)
Water 27.8% (v/v)
Propanediol 20.0% (v/v)
Ethoxy-glycol 1.5% (v/v)
Oleuropein (from dry extract of olive leaf) 1.2% (w/v)
Sodium bicarbonate 0.5% (w/v)
Glutathione / Vitamin F thioester 0.4% (w/v)
Lactic acid 0.2% (w/v)
Methyl nicotinate 0.05% (w/v)
this has been applied over an area of 5 cm² of scalp affected by incipient alopecia in the ratio of 0.1 mL per cm² for each of five male subjects, aged 35 to 55, with a diagnosis of androgenetic alopecia unaccompanied by psoriasis or seborrheic dermatitis, or with scars or atrophy or burns or local infections. The product was manually friction-rubbed in after each application, until it completely evaporated.

Another region of equal scalp area affected by incipient alopecia in every subject was treated in the same way, using a lotion of equivalent composition but without the Oleuropein (active preparation 2):
Ethanol 48.0% (v/v)
Water 28.2% (v/v)
Propanediol 20.0% (v/v)
Ethoxy-glycol 1.5% (v/v)
Sodium bicarbonate 0.5% (w/v)
Glutathione / Vitamin F thioester 0.4% (w/v)
Lactic acid 0.2% (w/v)
Methyl nicotinate 0.05% (w/v)
Another region of equal scalp area affected by incipient alopecia in every subject was treated in the same way, using a lotion of equivalent composition, but without the Glutathione/Vitamin F thioester (active compound 3):
Ethanol 48.0% (v/v)
Water 28.2% (v/v)
Propanediol 20.0% (v/v)
Ethoxy-glycol 1.5% (v/v)
Oleuropein (from dry extract of olive leaf) 1.2% (w/v)
Sodium bicarbonate 0.5% (w/v)
Lactic acid 0.2% (w/v)
Methyl nicotinate 0.05% (w/v)
Another region of equal scalp area affected by incipient alopecia in every subject was treated in the same way, using a lotion of equivalent composition, but without the Glutathione/Vitamin F thioester (control preparation 1):
Ethanol 49.0% (v/v)
Water 28.2% (v/v)
Propanediol 20.0% (v/v)
Ethoxy-glycol 1.5% (v/v)
Sodium bicarbonate 0.5% (w/v)
Lactic acid 0.2% (w/v)
Methyl nicotinate 0.05% (w/v)
At the end of twenty-one days (Checkpoint 1) and forty-two days (Checkpoint 2) of treatment being performed, the trichological examination was performed using photo-tricogrammetry on 2 cm² randomly chosen treated areas and control areas each. The results of the study are shown in the following table. The values are expressed as the mean of all measurements conducted.

| | Checkpoint 1 | | Checkpoint 2 | |
|---|---|---|---|---|
| | No. hairs/cm² | Average length (mm) | No. hairs/cm² | Average length (mm) |
| Active preparation 1 | 22 | 8 | 28 | 12 |
| Active preparation 2 | 10 | 3 | 7 | 4 |
| Active preparation 3 | 8 | 3 | 7 | 5 |
| Control preparation 1 | 5 | 3 | 5 | 4 |

### Example 2

A mask for hair, having the following composition (active preparation 4):
Water 55.5% (v/v)
Propanediol 20.0% (v/v)
EVOO 15% (w/v)
Hydrotalcites (HTlc) 4.0% (v/v)
Ethoxy-glycol 1.5% (v/v)
Oleuropein (from dry extract of olive leaf) 1.2% (w/v)
Sodium bicarbonate 0.5% (w/v)
Glutathione / Vitamin F thioester 0.4% (w/v)
Azelaic acid 0.3% (w / v)
Beta-sitosterol 0.3% (w / v)
Lactic acid 0.2% (w/v)
Zinc gluconate 0.05% (w / v)
Capsaicin 0.01% (w / v)
was applied on a circled area of about 25 cm² of scalp, in the region of 0.4 mL per cm² for each of six male subjects and four female subjects with thinning hair, between 30 and 45 years of age, in the absence of alopecia, psoriasis or seborrheic dermatitis, or scars or atrophy or burns or local infections. The product was uniformly distributed manually over the circled area of scalp, left to act for a period of between 20 and 25 minutes and finally rinsed away using plenty of water and a neutral shampoo.
A different circled area of scalp, of equal radius, on the opposite hemisphere of every subject was treated in the same way, using a mask of equal composition but without Glutathione/Vitamin F thioester (active preparation 5):
Water 55.5% (v/v)
Propanediol 20.0% (v/v)
EVOO 15% (w/v)
Hydrotalcites (HTlc) 4.0% (v/v)
Ethoxy-glycol 1.5% (v/v)
Oleuropein (from dry extract of olive leaf) 1.2% (w/v)
Sodium bicarbonate 0.5% (w/v)
Azelaic acid 0.3% (w / v)
Beta-sitosterol 0.3% (w / v)
Lactic acid 0.2% (w/v)
Zinc gluconate 0.05% (w / v)
Capsaicin 0.01% (w / v)
A different circled area of scalp, of equal radius in the opposite hemisphere of each subject was treated in the same way, using a mask of equal composition but without the Oleuropein (active preparation 6):
Water 55.5% (v/v)
Propanediol 20.0% (v/v)
EVOO 15% (w/v)
Hydrotalcites (HTlc) 4.0% (v/v)
Ethoxy-glycol 1.5% (v/v)
Sodium bicarbonate 0.5% (v / v)
Glutathione / Vitamin F thioester 0.4% (w / v)
Azelaic acid 0.3% (w/v)
Beta-sitosterol 0.3% (w / v)
Lactic acid 0.2% (w / v)
Zinc gluconate 0.05% (w / v)
Capsaicin 0.01% (w / v)
A different circled area of scalp, of equal radius in the same hemisphere of every subject was treated in the same way, using a mask of equal composition without Glutathione/Vitamin F thioester and Oleuropein (control preparation 2):
Water 55.5% (v/v)
Propanediol 20.0% (v/v)
EVOO 15% (w/v)
Hydrotalcites (HTlc) 4.0% (v/v)
Ethoxy-glycol 1.5% (v/v)
Sodium bicarbonate 0.5% (v/v)
Azelaic acid 0.3% (w/v)
Beta-sitosterol 0.3% (w/v)
Lactic acid 0.2% (w/v)
Zinc gluconate 0.05% (w/v)
Capsaicin 0.01% (w/v)
At the end of forty-five days of treatment, the trichological examination was carried out microscopically on about 60 hairs taken from each of the treated areas. The results are summarised in the following table. The values shown are the averages from all the subjects examined.

| | % in the Anagen phase | % in the Catagen phase | % in the Telogen phase | Average diameter, about 1 cm from the bulb (µm) |
|---|---|---|---|---|
| Active preparation 4 | 90.0 | 0.6 | 9.4 | 74.5 |
| Active preparation 5 | 86.2 | 1.7 | 12.1 | 66.9 |
| Active preparation 6 | 86.2 | 1.4 | 12.4 | 67.2 |
| Preparation for control 2 | 85.3 | 1.65 | 13.05 | 65 |

### Example 3

A mask having the composition of active preparation 4 was applied onto the surface of the scalp of No. 5 subjects (3 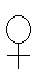 , 2 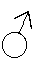 ) aged between 30 and 65 years, in the absence of alopecia, psoriasis or seborrheic dermatitis, or of scars or atrophies or burns or local infections, at 10 mL/day per subject, for 30 days. The product was distributed manually and left to act for about 20 minutes before being removed by washing with water and a neutral shampoo. In the 30 successive days the same subjects were treated with control preparation 2, in the same way.

At the beginning of the treatment, at the end of the 30 days, the 60 days and an additional period of 30 days of *washout, wash tests* were conducted on all subjects.

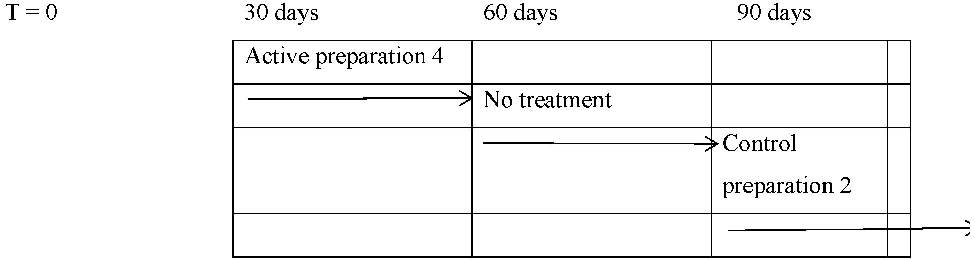

The results are summarised in the following table.

| | **T = 0** | **30 days** | **60 days** | **90 days** |
|---|---|---|---|---|
| No. hairs after washing (mean ± SD) | 85 ± 7 | 35 ± 6 | 68 ± 8 | 93 ± 6 |

### Example 4

The active preparation was applied over an area of 5 cm² of scalp affected by incipient alopecia at 0.1 mL per cm² for each of five male subjects, aged 35 to 55, with a diagnosis of androgenetic alopecia unaccompanied by psoriasis or seborrheic dermatitis, or with scars or atrophy or burns or local infections. The product was manually friction-rubbed in after each application, until it completely evaporated.
Another region of equal glabrous scalp surface area was treated in the same way, using the control preparation 1 in all subjects.
At the same time, eight of these subjects took, orally, every day, one capsule containing a preparation that had the following composition:
Oleuropein (from dry extract of olive leaf) 120 mg
Glutathione conjugated with Vitamin F thioester 200 mg
Excipients (hydroxy-methylcellulose, silica) q.b.
At the end of forty-two days of treatment, a trichological examination was performed using photo-tricogrammetry on 2 cm² randomly chosen from each of the treated areas and control areas. The results of the study are shown in the following table. The values are expressed as the mean of all measurements conducted.

| | No. hairs/cm² | Average length (mm) |
|---|---|---|
| Subjects treated only topically | 26 | 11 |
| Subjects treated via topical and systemic routes | 36 | 13 |
| Control preparation 1 | 5 | 4 |

## Claims

1. Pharmaceutical composition for use in a therapeutic method of counteracting hair loss and of promoting hair growth in humans and in other species of mammals comprising, as active ingredients, glutathione thioester and at least one secoiridoid of *Olea europea* in the form of glycoside or aglycone.

2. Composition for use according to claim 1, wherein the glutathione thioester is one or more S-acyl derivatives of glutathione having chemical formula 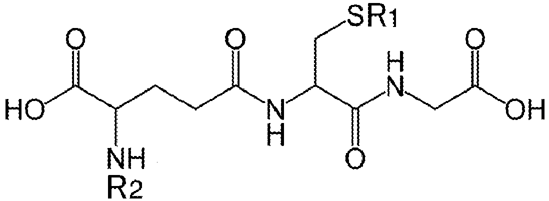 where R1 is a carboxylic acid residue with 2-22 carbon atoms, saturated or unsaturated, and R2 is a hydrogen atom or a carboxylic acid residue with 2-22 carbon atoms, saturated or unsaturated.

3. Composition for use according to claim 1 or 2, wherein the secoiridoid of *Olea europea* in the glycosidic form is glycosylated oleuropein, an organic compound represented by chemical formula

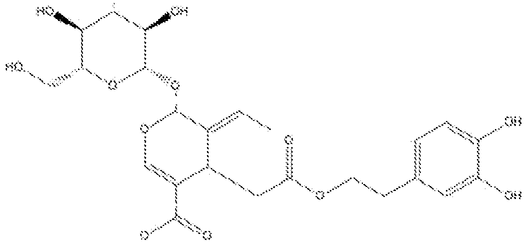

4. Composition for use according to claim 1 or 2, wherein the secoiridoid of *Olea europea* in the aglycone form is oleuropein aglycone, an organic compound represented by the chemical formula

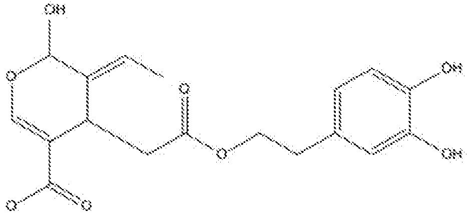

5. Composition for use according to claim 1 or 2 comprising, as secoiridoid of *Olea europea,* one or more metabolites of oleuropein, such as tyrosol or hydroxytyrosol, or other secoiridoid of *Olea europea,* such as oleuroside, elenoic acid, ligstroside and nuzhenide.

6. Composition for use as claimed in any one of the preceding claims, wherein the secoiridoid of *Olea europea* is provided by a dried hydroalcoholic extract of *Olea europea* tissues, preferably from *Olea europea* leaves.

7. Composition for use as claimed in any one of the preceding claims, wherein glutathione thioester is present in an amount from 1 mg to 500 mg per gram, preferably between 5 mg and 200 mg per g, and the secoiridoid of *Olea europea* is present in quantities between 1 and 350 mg per g, preferably between 10 and 100 mg per g.

8. Composition for use as claimed in any one of the preceding claims containing, in addition to glutathione thioester and to one or more secoiridoid of *Olea europea,* other substances useful for the purposes of its effectiveness or administration or storage such as a carrier, an antioxidant, a flavoring agent, a pigment, one or more micronutrients, an emulsifier, a solvent, a stabilizer.

9. A cosmetic method for preventing or treating hair loss or promoting hair growth by means of a composition according to any one of the preceding claims, wherein said composition, in liquid or solid form, is a pharmaceutical remedy, a cosmetic product, a medical device, a nutraceutical preparation or a functional food.

10. A cosmetic method according to claim 9, wherein the composition is administered orally, parenterally, topically, by means of micro-intradermal injections, or by means of iontophoresis.

11. A cosmetic method according to claim 9 or 10, wherein the very two active ingredients are administered simultaneously at both topical and systemic levels, by the use of a hair lotion and a nutritional supplement.

## Patentansprüche

1. Pharmazeutische Zusammensetzung zur Verwendung in einem therapeutischen Verfahren, um beim Menschen und anderen Säugetierspezies Haarverlust entgegenzuwirken und Haarwachstum anzuregen, umfassend, als Wirkstoffe, Glutathionthioester und mindestens ein Secoiridoid von *Olea europea* in der Form von Glycosid oder Aglycon.

2. Zusammensetzung zur Verwendung nach Anspruch 1, wobei der Glutathionthioester ein oder mehrere S-Acylderivate von Glutathoin ist mit der chemischen Formel 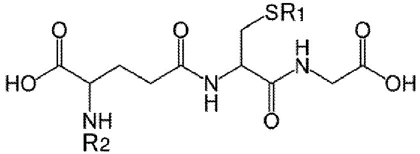 wo R1 ein Carbonsäurerest mit 2-22 Kohlenstoffatomen, gesättigt oder ungesättigt, ist und R2 ein Wasserstoffatom oder ein Carbonsäurerest mit 2-22 Kohlenstoffatomen, gesättigt oder ungesättigt, ist.

3. Zusammensetzung zur Verwendung nach Anspruch 1 oder 2, wobei das Secoiridoid von *Olea europea* in der Glycosidform glycosylatiertes Oleuropein ist, eine organische Verbindung, die durch die chemische Formel dargestellt ist

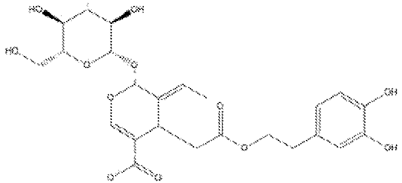

4. Zusammensetzung zur Verwendung nach Anspruch 1 oder 2, wobei das Secoiridoid von *Olea europea* in der Aglyconform Oleuropeinaglycon ist, eine organische Verbindung, die durch die chemische Formel dargestellt ist

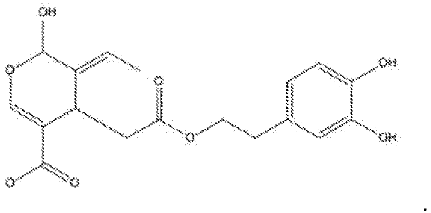

5. Zusammensetzung zur Verwendung nach Anspruch 1 oder 2, umfassend, als Secoiridoid von *Olea europea,* ein oder mehr Metaboliten von Oeuropein, wie Tyrosol oder Hydroxytyrosol, oder ein anderes Secoiridoid von *Olea europea,* wie Oleurosid, Elenolsäure, Ligstrosid und Nuzhenid.

6. Zusammensetzung zur Verwendung nach einem der vorstehenden Ansprüche, wobei das Secoiridoid von *Olea europea* durch ein getrocknetes Hydroalkoholextrakt von *Olea europea-Geweben* bereitgestellt ist, bevorzugt von *Olea europea* Blättern.

7. Zusammensetzung zur Verwendung nach einem der vorstehenden Ansprüche, wobei Glutathointhioester in einer Menge von 1 mg bis 500 mg pro Gramm vorhanden ist, bevorzugt zwischen 5 mg und 200 mg pro g, und das Secoiridoid von *Olea europea* in Mengen zwischen 1 und 350 mg pro g vorhanden ist, bevorzugt zwischen 10 und 100 mg pro g.

8. Zusammensetzung zur Verwendung nach einem der vorstehenden Ansprüche, die zusätzlich zu Glutathointhioester und zu einem oder mehr Secoiridoid von *Olea europea* andere Substanzen enthält, die für die Zwecke ihrer Effektivität oder Verabreichung oder Lagerung nützlich ist, wie einen Trägerstoff, ein Antioxidans, ein Geschmacksmittel, ein Pigment, eine oder mehrere Mikronährstoffe, einen Emulgator, ein Lösemittel, einen Stabilisator.

9. Kosmetikverfahren zum Verhindern oder Behandeln von Haarverlust oder Anregen von Haarwachstum mittels einer Zusammensetzung nach einem der vorstehenden Ansprüche, wobei die Zusammensetzung in flüssiger oder fester Form ein pharmazeutisches Arzneimittel, ein Kosmetikprodukt, eine medizinische Vorrichtung, ein nutrazeutisches Präparat oder ein funktionelles Lebensmittel ist.

10. Kosmetikverfahren nach Anspruch 9, wobei die Zusammensetzung oral, parenteral, topikal, mittels mikrointrakutaner Injektionen oder mittels lontophorese verabreicht wird.

11. Kosmetikverfahren nach Anspruch 9 oder 10, wobei die genau zwei Wirkstoffe gleichzeitig sowohl auf topikaler als auch systemischer Ebene durch die Verwendung einer Haarlotion und eines Ernährungsergänzungsmittels verabreicht werden.

## Revendications

1. Composition pharmaceutique pour son utilisation dans un procédé thérapeutique pour neutraliser la perte de cheveux et stimuler la croissance des cheveux chez les êtres humains et chez d'autres espèces de mammifères comprenant, en tant que principes actifs, un thioester de glutathion et au moins un sécoiridoïde d'*Olea europea* sous la forme d'un glycoside ou d'un aglycone.

2. Composition pour son utilisation selon la revendication 1, dans laquelle le thioester de glutathion est un ou plusieurs dérivés S-acyle de glutathion ayant la formule chimique 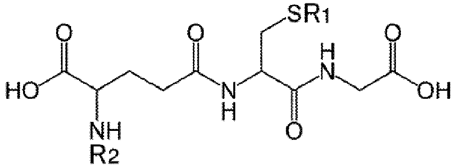 dans laquelle R1 est un résidu d'acide carboxylique avec 2 à 22 atomes de carbone, saturé ou insaturé, et R2 est un atome d'hydrogène ou un résidu d'acide carboxylique avec 2 à 22 atomes de carbone, saturé ou insaturé.

3. Composition pour son utilisation selon la revendication 1 ou 2, dans laquelle le sécoiridoïde d'*Olea europea* sous la forme glycosidique est l'oleuropéine glycosylée, un composé organique représenté par la formule chimique

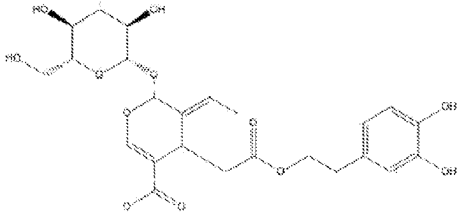

4. Composition pour son utilisation selon la revendication 1 ou 2, dans laquelle le sécoiridoïde d'*Olea europea* sous la forme aglycone est un aglycone d'oleuropéine, un composé organique représenté par la formule chimique

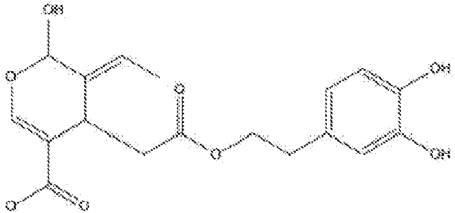

5. Composition pour son utilisation selon la revendication 1 ou 2 comprenant, en tant que sécoiridoïde d'*Olea europea,* un ou plusieurs métabolites d'oleuropéine, comme le tyrosol ou l'hydroxytyrosol, ou un autre sécoiridoïde d'*Olea europea,* comme l'oleuroside, l'acide élénoïque, le ligstroside et le nuzhénide.

6. Composition pour son utilisation selon l'une quelconque des revendications précédentes, dans laquelle le sécoiridoïde d'*Olea europea* est fourni par un extrait hydroalcoolique sec de tissus d'*Olea europea,* de préférence de feuilles d'*Olea europea.*

7. Composition pour son utilisation selon l'une quelconque des revendications précédentes, dans laquelle le thioester de glutathion est présent en une quantité de 1 mg à 500 mg, de préférence de 5 mg à 200 mg par g, et le sécoiridoïde d'*Olea europea* est présent en des quantités comprises entre 1 et 350 mg par g, de préférence entre 10 et 100 mg par g.

8. Composition pour son utilisation selon l'une quelconque des revendications précédentes contenant, outre le thioester de glutathion et un ou plusieurs sécoiridoïdes d'*Olea europea,* d'autres substances utiles pour les fins de son efficacité ou de son administration ou de son stockage sous la forme d'un vecteur, d'un antioxydant, d'un agent aromatisant, d'un pigment, d'un ou plusieurs micronutriments, d'un émulsifiant, d'un solvant, d'un stabilisant.

9. Procédé cosmétique de prévention ou de traitement de la perte de cheveux ou de stimulation de la croissance des cheveux au moyen d'une composition selon l'une quelconque des revendications précédentes, dans lequel ladite composition, sous une forme liquide ou solide, est un remède pharmaceutique, un produit cosmétique, un dispositif médical, une préparation nutraceutique, ou un aliment fonctionnel.

10. Procédé cosmétique selon la revendication 9, dans lequel la composition est administrée par voie orale, parentérale, topique, au moyen de micro-injections intradermiques, ou par ionophorèse.

11. Procédé cosmétique selon la revendication 9 ou 10, dans lequel les deux principes actifs sont administrés simultanément aux niveaux topique et systémique, à l'aide d'une lotion capillaire et d'un supplément nutritionnel.
